# NEUE EUROPÄISCHE PATENTSCHRIFT

(11) **EP 0 071 965 B2**
(45) Veröffentlichungstag und Bekanntmachung des Hinweises auf die Entscheidung über den Einspruch: **07.04.1993**
(45) Hinweis auf die Patenterteilung: 01.04.1987
(21) Anmeldenummer: 82107009.1
(22) Anmeldetag: 03.08.1982
(51) Int. Cl.: A61N 1/362

(54) **Implantierbarer Herzschrittmacher (Sicherstellung einer Minimalamplitude)**
Implantable heart pacemaker (ensuring a minimal amplitude)
Stimulateur cardiaque implantable (sûreté d'une amplitude minimale)

(30) Priorität: 04.08.1981 DE 3130872
(43) Veröffentlichungstag der Anmeldung: 16.02.1983
(73) Patentinhaber: Siemens-Elema AB, 171 95 Solna 1 (SE); SIEMENS AKTIENGESELLSCHAFT, 80333 München (DE)
(72) Erfinder: Elmqvist, Hakan, Dr., S-16138 Bromma (SE)

(56) Entgegenhaltungen:
- EP-A- 0 017 608
- EP-A- 0 029 479
- DE-A- 2 301 055
- DE-A- 2 342 030
- DE-A- 2 702 517
- DE-B- 1 296 283
- US-A- 4 120 307

## Beschreibung

Die Erfindung betrifft einen implantierbaren Herzschrittmacher mit einer Batterie, deren Innenwiderstand vom Batterieladezustand abhängt, sowie einem an die Batterie angeschlossenen Reizimpulsgenerator mit einem Ausgangskondensator, der jeweils langsam aufgeladen und anschließend zur Erzeugung eines Stimulierungsimpulses schnell entladen wird, wobei die Entladung des Ausgangskondensators erst dann auslösbar ist, wenn er mindestens bis zu einer bestimmten Mindestspannung aufgeladen ist.

Derartige moderne implantierbare Herzschrittmacher verwenden Batterien mit konstanter elektromotorischer Kraft, deren innerer Widerstand ansteigt, je stärker die Batterie entladen wird. Das führt dazu, daß die Amplitude der Stimulierungsimpulse mit zunehmender Entladung der Batterie abnimmt, da der Ausgangskondensator mit einem immer kleiner werdenden Strom aufgeladen werden muß und somit in der für die Aufladung zur Verfügung stehenden Zeit nicht mehr seine volle Spannung erreicht. Wenn die Amplitude der Stimulierungsimpulse unter den für den Patienten charakteristischen Schwellwert absinkt, werden die Stimulierungsimpulse ineffektiv.

Es sind bereits Versuche unternommen worden, die Betriebsdauer eines derartigen Herzschrittmachers zu verlängern, indem die Breite der Stimulierungsimpulse vergrößert wird, wenn sich die Batterie entlädt (DE-A-2 301 055). Da sich jedoch der Schwellwert nur in ganz geringem Maße durch die Breite der Stimulierungsimpulse beeinflussen lässt, ist auf diesem Wege nur eine unbedeutende Verlängerung der Lebensdauer erzielbar. Darüber hinaus ergibt sich bei dieser Methode der Nachteil eines erhöhten Stromverbrauches pro Impuls und damit einer beschleunigten Entladung der Batterie.

Aus der DE-B-1 296 283 ist ein externer Herzschrittmacher mit einem an einer Anodenbatterie angeschlossenen Reizimpulsgenerator bekannt, bei dem ein Kondensator mit einer dazu parallel geschalteten selbstzündenden gasgefüllten Röhre in Reihe mit einem Widerstand an der Anodenbatterie angeschlossen ist. Sobald die Spannung an dem Kondensator den Wert für die Zündspannung der Röhre erreicht, wird der Kondensator automatisch über die Röhre entladen, wobei mittels eines Transformators aus dem Entladestrom ein Stimulierungsimpuls für das Herz erzeugt wird. Die Amplitude der Stimulierungsimpulse ist auf Grund der konstanten Zündspannung der Röhre vom jeweiligen Entladezustand der Anodenbatterie unabhängig. In erster Näherung ist dabei zumindest für eine bestimmte Zeit, solange die Anodenbatterie im wesentlichen voll aufgeladen ist, eine konstante Stimulationsfrequenz gegeben. Wenn der Entladezustand jedoch soweit fortgeschritten ist, daß diese erste Näherung nicht mehr gilt, nimmt die Stimulationsfrequenz mit zunehmender Entladung der Batterie ab. Außerdem ist die Stimulationsfrequenz ausschließlich über den Wert des Ausgangskondensators und die Werte der in seinem Aufladezweig liegenden Widerstände bestimmt. Eine unabhängig von diesem Ladevorgang einstellbare Stimulationsfrequenz ist mit dieser Schaltung nicht möglich. Weiterhin ist es mit dieser Schaltung nicht möglich, die Stimulationsfrequenz auch bei einem Entladezustand der Batterie konstant zu halten, für die die oben genannte erste Näherung nicht mehr zutrifft.

Der Erfindung liegt die Aufgabe zugrunde, einen Herzschrittmacher anzugeben, der stets eine gewisse Mindestamplitude der Stimulierungsimpulse füreine sichere Reizung des Herzens zur Verfügung stellt, wobei aber gleichzeitig die Stimulationsfrequenz, solange es der Ladezustand der Batterie zuläßt, von diesem unabhängig ist. Ferner soll ein Anzeichen für den kritisch werdenden Zustand der Batterie geliefert werden.

Gemäß der Erfindung wird diese Aufgabe dadurch gelöst, daß bei dem Herzschrittmacher der eingangs angegebenen Art die Entladung des Ausgangskondensators erfolgt, wenn zusätzlich eine über ein unabhängig vom Ladevorgang arbeitendes Zeitglied definierte Schaltzeit abgelaufen ist, die die Grundfrequenz des Herzschrittmachers festlegt, und daß bei der Entladung des Ausgangskondensators das Zeitglied jeweils neu gestartet wird. Der Herzschrittmacher gibt dadurch Impulse ab, die zumindest die eingestellte Mindestamplitude aufweisen, deren Frequenz aber unabhängig davon durch das Zeitglied festgelegt ist. Da diese oberhalb des Schwellwertes liegen, wird das Herz sicher stimuliert. Handelt es sich beispielsweise um einen Herzschrittmacher, der mit einer bestimmten einstellbaren Grundfrequenz arbeitet, so kann diese Grundfrequenz aufrechterhalten werden, bis die dadurch festgelegte Aufladezeit bei steigendem Innenwiderstand der Batterie nicht mehr ausreicht, um den Ausgangskondensator auf die festgesetzte Minstestspannung aufzuladen. Die Frequenz beginnt dann mit steigendem Batterieinnenwiderstand derart zu sinken, daß die Amplitude der Ausgangsimpulse konstant dem Mindestwert entspricht. Dadurch kann für jeden Impuls die Mindestamplitude sichergestellt werden.

In einer vorteilhaften Weiterbildung der Erfindung ist vorgesehen, dass die Mindestspannung Vmm einstellbar ist. Insbesondere bei modernen programmierbaren Herzschrittmachern sollte auch diese Spannung programmierbar sein. Die Regelung des Impulsgenerators kann sowohl analog als auch digital erfolgen. Bevorzugte Ausgestaltungen der Erfindung sind in den Unteransprüchen gekennzeichnet.

Da bei dem erfindungsgemässen Herzschrittmacher die Frequenz bis zu einem gewissen Grenzwert des Batterieinnenwiderstandes konstant bleibt und dann anfängt, kontinuierlich zu sinken, stellt der Knickpunkt in der Frequenzkurve eine sichere Marke für den Batterieladezustand dar.

Eine vorteilhaft einfache und sichere Ausführungsform eines erfindungsgemässen Herzschrittmachers mit einem bekannten Kurzschlussschalter, insbesondere einem Transistorschalter, ergibt sich dadurch, dass der Schalter durch eine monostabile Kippstufe betätigbar ist, deren Schaltzeit tₘ im Bereich der gewünschten Entladezeitdauer liegt. Der Eingang dieser monostabilen Kippstufe ist mit dem Ausgang eines NOR-Gatters verbunden, dessen einer Eingang mit dem Ausgang des weitere monostabile Kippstufe mit einer eine Grundfrequenz des Herzschrittmachers festlegenden Schaltzeit ausgebildeten Zeitgliedes und dessen anderer Eingang mit dem Ausgang eines Komparators verbunden ist. Der Ausgang der ersten monostabilen Kippstufe ist weiterhin mit dem Eingang der weiteren monostabilen Kippstufe verbunden. An dem einen Eingang des Komparators liegt die Mindestspannung Vmm und an dem anderen Eingang die Spannung des Ausgangskondensators an. Die weitere monostabile Kippstufe legt dabei die Grundfrequenz und dadurch auch die Aufladezeit für den Ausgangskondensator fest. Erreicht die Spannung am Ausgangskondensator in dieser Zeit die Mindestspannung Vmm oder übersteigt diese sogar, so wird am Ende der Schaltzeit der weiteren monostabilen Kippstufe die erste monostabile Kippstufe angesteuert und derAusgangskondensator für eine kurze Zeitdauer kurzgeschlossen. Erreicht hingegen die Ladespannung am Ausgangskondensator in der vorgegebenen Schaltzeit nicht die Mindestspannung, so wird die erste monostabile Kippstufe und damit die Kurzschliessung des Ausgangskondensators blockiert, bis die Spannung den Minimalwert erreicht hat. Die Amplitude der Stimulierungsimpulse kann daher niemals unter die Mindestspannung Vmm absinken. Gleichzeitig nimmt bei sinkender Frequenz der Stromverbrauch pro Zeiteinheit im Gegensatz zu bekannten Herzschrittmachern, bei denen bei erhöhtem Batterieinnenwiderstand die Impulsbreite der Stimulierungsimpulse vergrössert wird, ab. Ein weiterer Vorteil des erfindungsgemässen Herzschrittmachers besteht in einem gewissen Schutz gegen eine unkontrollierte Erhöhung der Herzschrittmacherfrequenz durch einen auftretenden Fehler. Die Impulse können stets erst dann abgegeben werden, wenn die Spannung am Kondensator die Mindestspannung übersteigt.

An Hand zweier Figuren ist im folgenden ein Ausführungsbeispiel der Erfindung näher beschrieben und erläutert.

Dabei zeigt
Fig. 1 in einem Blockschaltbild die wesentlichsten Teile zur Verdeutlichung des erfindungsgemässen Herzschrittmachers, und
Fig. 2 den zeitlichen Verlauf verschiedener Kenngrössen, wie z.B. der Frequenz.

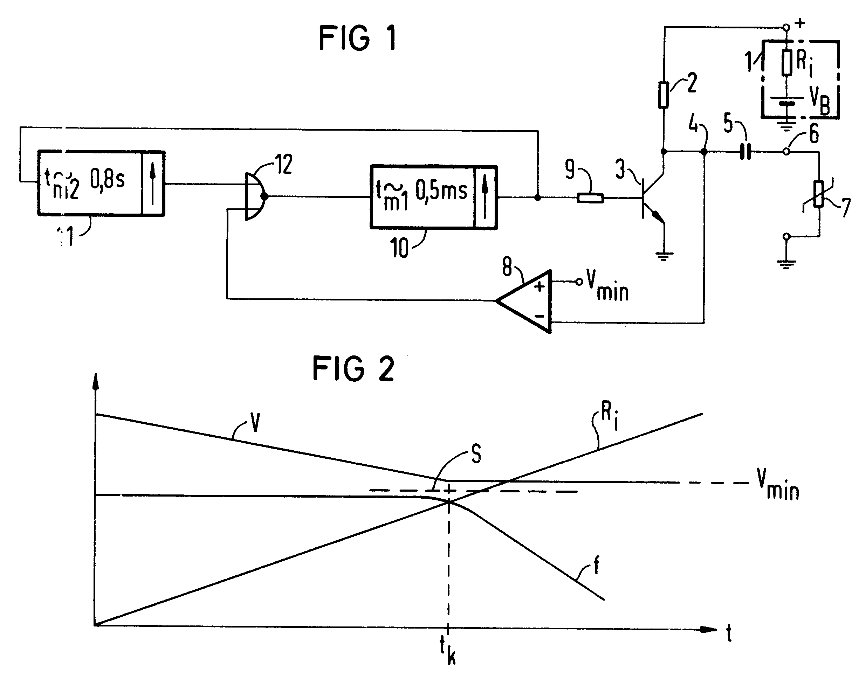

In Fig. 1 ist gestrichelt eine Batterie 1 mit einem vom Ladezustand abhängigen Innenwiderstand Rᵢ dargestellt. Die Klemmspannung der Batterie ist mit V_{B} bezeichnet. Ein Pol der Batterie liegt auf Masse, d. h. er ist mit dem Körper eines Patienten verbunden. Der andere Pol ist über einen festen Widerstand 2 mit dem Kollektor eines Transistors 3 und einem Anschluss 4 des Ausgangskondensators 5 verbunden. Der andere Anschluss 6 des Kondensators 5 ist über eine Elektrode mit dem Herzen des Patienten, hier als veränderbarer Widerstand 7 dargestellt, verbunden. Der Stromkreis ist über den Körper des Patienten geschlossen. Weiterhin ist der Anschluss 4 mit dem Minuseingang eines Komparators 8 verbunden, an dessen Pluseingang eine Vergleichsspannung Vₘᵢₙ angelegt ist. Der Emitter des Transistors 3 ist mit Masse verbunden; die Basis über einen Widerstand 9 mit dem Ausgang einer monostabilen Kippstufe 10 mit einer Schaltzeit tₘ₁ von etwa 0,5 ms. Der Ausgang dieser monostabilen Kippstufe 10 ist gleichzeitig mit dem Eingang einer weiteren monostabilen Kippstufe 11 mit einer Schaltzeit tₘ₂ von etwa 0,8 s verbunden. Die Ausgänge dieser weiteren monostabilen Kippstufe 11 und des Komparators 8 sind an die Eingänge eines NOR-Gatters 12 angeschlossen, dessen Ausgang die erste monostabile Kippstufe 10 ansteuert. Die Funktionsweise dieser Schaltung ist folgende:

Die beiden monostabilen Kippstufen befinden sich in ihrer Anfangsstellung, d. h. die Ausgänge liegen auf niedrigem Potential. Dadurch ist der Transistor 3 gesperrt und der Kondensator 5 lädt sich über den Widerstand 2 langsam auf. Solange die Spannung am Kondensator 5 noch nicht die Vergleichsspannung Vmm erreicht hat, liegt der Ausgang des Komparators 8 auf hohem Potential. Dadurch liegen an den Eingängen des NOR-Gatters 12 verschiedene Potentiale und der Ausgangswert dieses Gatters liegt auf Null. Erreicht die Spannung am Kondensator 5 die Vergleichsspannung Vₘᵢₙ, so wird die Ausgangsspannung des Komparators 8 niedrig, so dass an beiden Eingängen des NOR-Gatters eine logische Null liegt. Der Ausgang des NOR-Gatters wird damit logisch eins, wodurch die monostabile Kippstufe für die Schaltzeit tₘ₁ in ihre Arbeitslage übergeht. Für diese Zeitweistder Ausgang dieser monostabilen Kippstufe 10 ein hohes Potential auf, wodurch der Transistor 3 durchgesteuert wird. Der Kondensator 5 kann sich schnell über den Transistor 3 entladen, wodurch dem Herzen ein Stimulierungsimpuls zugeführt wird. Gleichzeitig wird beim Umschalten der monostabilen Kippstufe 10 die weitere monostabile Kippstufe 11 gestartet, wodurch deren Ausgang für die Schaltzeit tₘ₂ eine logische Eins annimmt. Das NOR-Gatter 12 wird damit wieder gesperrt.

Solange der Innenwiderstand Rᵢ noch so klein ist, dass die Spannung am Kondensator 5 in der Schaltzeit tₘ₂ der monostabilen Kippstufe 11 die Vergleichsspannung Vₘᵢₙ erreicht und sogar übersteigt, wird die Frequenz der Stimulierungsimpulse durch die Schaltzeit dieser monostabilen Kippstufe 11 festgesetzt. Ist der Innenwiderstand Rᵢ der Batterie 1 so weit angewachsen, dass die Spannung am Kondensator 5 in der Schaltzeit tₘ₂ die Vergleichsspannung Vₘᵢₙ noch nicht erreicht hat, so sperrt das NOR-Gatter 12 bis zum Erreichen der Vergleichsspannung. Erst dann wird die monostabile Kippstufe 10 gestartet und der Kondensator 3 durchgeschaltet. Die Frequenz der Stimulierungsimpulse nimmt daher von dem Zeitpunkt an mit wachsendem Innenwiderstand Rᵢ der Batterie 1 ab.

Zur Änderung der Grundfrequenz des Herzschrittmachers kann die Schaltzeit tₘ₂ der monostabilen Kippstufe 11 veränderbar sein. Ebenso kann die Vergleichsspannung Vₘᵢₙ veränderbar ausgebildet sein. Diese und andere Grössen können vorteilhafterweise programmierbar sein.

In Fig. 2 ist in willkürlichem Massstab in einem Diagramm der zeitliche Verlauf der Frequenz des Herzschrittmachers, der Amplitude der Stimulierungsimpulse sowie der Innenwiderstand der Batterie dargestellt. Wie man diesem Diagramm entnehmen kann, steigt der Innenwiderstand Rᵢ der Batterie 1 linear mit der Zeit an. Gleichzeitig sinkt die Amplitude der Stimulierungsimpulse ab. Solange diese Amplitude aber noch oberhalb der Mindestspannung Vₘᵢₙ liegt, bleibt die Frequenz des Herzschrittmachers unverändert. Zu einem bestimmten Zeitpunkt tₖ würde die Amplitude üblicherweise die Mindestspannung Vₘᵢₙ unterschreiten, so dass die sichere Stimulierung des Herzens durch den Stimulierungsimpuls nicht mehr gewährleistet wäre. Durch die erfindungsgemässe Schaltungsanordnung beginnt zu diesem Zeitpunkt die Frequenz der Stimulierungsimpulse derart abzunehmen, dass die Spannung am Kondensator 5 und damit die Amplitude der Stimulierungsimpulse konstant der Mindestspannung Vₘᵢₙ entspricht. Weiterhin ist in diesem Diagramm gestrichelt der Schwellwert S des Herzens eingezeichnet, um anzudeuten, dass die Amplitude der Stimulierungsimpulse stets grösser ist als der Schwellwert.

## Patentansprüche

1. Implantierbarer Herzschrittmacher mit einer Batterie (1), deren Innenwiderstand (Rᵢ) vom Batterieladezustand abhängt, sowie einem an die Batterie angeschlossenen Reizimpulsgenerator mit einem Ausgangskondensator (5), der jeweils langsam aufgeladen und anschließend zur Erzeugung eines Stimulierungsimpulses schnell entladen wird, wobei die Entladung des Ausgangskondensators (5) erst dann auslösbar ist, wenn er mindestens bis zu einer bestimmten Mindestspannung (VmiO aufgeladen ist, dadurch gekennzeichnet, daß die Entladung des Ausgangskondensators erfolgt, wenn zusätzlich eine über ein unabhängig vom Ladevorgang arbeitendes Zeitglied (11) definierte Schaltzeit (tₘ₂) abgelaufen ist, die die Grundfrequenz des Herzschrittmachers festlegt, und daß bei der Entladung des Ausganskondensators (5) das Zeitglied (11) jeweils neu gestartet wird.

2. Herzschrittmacher nach Anspruch 1, dadurch gekennzeichnet, daß die Mindestspannung (Vₘᵢₙ) einstellbar, insbesondere programmierbar ist.

3. Herzschrittmacher nach Anspruch 1 oder 2, dadurch gekennzeichnet, daß die Spannung am Ausgangskondensator (5) analog erfaßt und über einen Analog-Digital-Wandler in ein digitales Signal umgewandelt wird, das mit digitalen Mitteln mit einem der Mindestspannung (Vₘᵢₙ) entsprechenden Digitalsignal verglichen wird.

4. Herzschrittmacher nach Anspruch 1 oder 2 mit einem bekannten Kurzschlußschalter, insbesondere einem Transistorschalter, zum Entladen des Ausgangskondensators, dadurch gekennzeichnet, daß der Schalter (3) durch eine monostabile Kippstufe (10) betätigbar ist, deren Schaltzeit (tₘᵢ) im Bereich der gewünschten Entladezeitdauer liegt, daß der Eingang dieser monostabilen Kippstufe (10) mit dem Ausgang eines NOR-Gatters (12) verbunden ist, dessen einer Eingang mit dem Ausgang des als weitere monostabile Kippstufe (11) mit der die Grundfrequenz des Herzschrittmachers festlegenden Schaltzeit (tₘ₂) ausgebildeten Zeitgliedes und dessen anderer Eingang mit dem Ausgang eines Komparators (8) verbunden ist, daß der Ausgang der ersten monostabilen Kippstufe (10) mit dem Eingang der weiteren monostabilen Kippstufe (11) verbunden ist und daß am einen Eingang des Komparators (8) die Mindestspannung (Vₘᵢₙ) und am anderen Eingang die Spannung des Ausgangskondensators (5) anliegt.

## Claims

1. Implantable pacemaker having a battery (1) whose internal resistance (Rᵢ) depends on the battery charge, and a stimulating pulse enerator which is connected to the battery, comprising an output capacitor (5) which is respectively slowly recharged and subsequently rapidly discharged in order to generate a stimulating pulse, it not being possible to trigger the discharge of the output capacitor (5) until it is recharged at least to a specific minimum voltage (Vₘᵢₙ), characterised in that the discharge of the output capacitor is per- formed when, in addition, a switching time (tₘ₂) has elapsed which is defined via a timing element (11) operating independently of the charging process and which fixes the fundamental frequency of the pacemaker, and in that the timing element (11) is started anew in each case upon discharge of the output capacitor (5).

2. Pacemaker according to Claim 1, characterised in that the minimum voltage (Vₘᵢₙ) is adjustable, in particular programmable.

3. Pacemaker according to Claim 1 or 2, characterised in that the voltage at the output capacitor (5) is detected in analog fashion and converted via an analog-to-digital converter into a digital signal which is compared using digital means to a digital signal corresponding to the minimum voltage (Vmn).

4. Pacemaker according to Claim 1 or 2 having a known short-circuiting switch, in particular a transistor switch, for discharging the output capacitor, characterised in that the switch (3) can be operated by a monostable multivibrator (10) whose switching time (tₘᵢ) is in the region of the desired discharge period, in that the input of this monostable multivibrator (10) is connected to the output of a NOR gate (12) whose one input is connected to the output of the timing element constructed as a further monostable multivibrator (11) with the switching time (tₘ₂) which fixes the fundamental frequency of the pacemaker, and whose other input is connected to the output of a comparator (8), in that the output of the first monostable multivibrator (10) is connected to the input of the further monostable multivibrator (11), and in that the minimum voltage (Vₘᵢₙ) is connected to the one input of the comparator (8) and the voltage of the output capacitor (5) is connected to the other input.

## Revendications

1. Stimulateur cardiaque implantable, comportant une pile (1) dont la résistance interne (Rᵢ) dépend de l'état de la charge de cette pile, ainsi qu'un générateur d'impulsions de stimulation, raccordé à la pile et comportant un condensateur de sortie (5), qui est chargé lentement et est ensuite déchargé rapidement pour produire une impulsion de stimulation, la décharge du condensateur de sortie (5) étant susceptible d'être déclenchée uniquement lorsque le condensateur est chargé à une tension minimale déterminée (Vₘᵢₙ), caractérisé par le fait que la décharge du condensateur de sortie s'effectue lorsque, une durée de commutation supplémentaire (tₘ₂), qui est définie par un circuit de temporisation (11) travaillant indépendamment du processus de charge et qui fixe la fréquence de base du stimulateur cardiaque, est écoulée, et que lors de la décharge du condensateur de sortie (5), le circuit de temporisation (11) est à nouveau déclenché.

2. Stimulateur cardiaque selon la revendication 1, caractérisé par le fait que la tension minimale (Vₘᵢₙ) est réglable et notamment est programmable.

3. Stimulateur cardiaque selon la revendication 1 ou 2, caractérisé par le fait que la tension aux bornes du condensateur de sortie (5) est détectée de façon analogique et est transformée, par l'intermédiaire d'un convertisseur analogique/numérique, en un signal numérique qui est comparé à l'aide de moyens numériques à un signal numérique correspondant à la tension minimale (Vmm).

4. Stimulateur cardiaque suivant la revendication 1 ou 2, comportant un commutateur de court- circuit connu, notamment un commutateur à transistors, pour décharger le condensateur de sortie, caractérisé par le fait que le commutateur (3) peut être actionné au moyen d'un étage à bascule monostable (10), dont la durée de commutation (tₘᵢ) est de l'ordre de la durée désirée de la décharge, que l'entrée de cet étage à bascule monostable (10) est reliée à la sortie d'une porte NON-OU (12), dont une entrée est raccordée à la sortie du circuit de temporisation agencé sous la forme d'un autre étage à bascule monostable (11) présentant la durée de commutation (tₘ₂) déterminant la fréquence limite du stimulateur cardiaque et dont l'autre entrée est reliée à la sortie d'un comparateur (8), que la sortie du premier étage à bascule monostable (10) est reliée à l'entrée depuis l'autre étage à bascule monostable (11) et que la tension minimale (Vₘᵢₙ) est appliquée à une entrée du comparateur (8) et que la tension du condensateur de sortie (5) est appliquée à l'autre entrée.
